(19) Europäisches Patentamt
European Patent Office
Office européen des brevets

(11) EP 1 037 648 B1

(12) **EUROPÄISCHE PATENTSCHRIFT**

(45) Veröffentlichungstag und Bekanntmachung des Hinweises auf die Patenterteilung:
**07.04.2004 Patentblatt 2004/15**

(21) Anmeldenummer: **98943842.9**

(22) Anmeldetag: **05.08.1998**

(51) Int Cl.⁷: **A61K 38/18**, A61K 33/26,
A61P 29/00
// (A61K38/18, 33:26)

(86) Internationale Anmeldenummer:
**PCT/EP1998/004864**

(87) Internationale Veröffentlichungsnummer:
**WO 1999/007401 (18.02.1999 Gazette 1999/07)**

(54) **VERWENDUNG VON ERYTHROPOIETIN UND EISENPRÄPARATEN ZUR HERSTELLUNG VON PHARMAZEUTISCHEN KOMBINATIONSPRÄPARATEN ZUR BEHANDLUNG VON RHEUMATISCHEN ERKRANKUNGEN**

THE USE OF ERYTHROPOIETIN AND IRON PREPARATIONS FOR PRODUCING PHARMACEUTICAL COMBINATION PREPARATIONS FOR TREATING RHEUMATIC DISEASES

UTILISATION DE PREPARATIONS D'ERYTHROPOIETINE ET DE FER POUR LA PRODUCTION DE PREPARATIONS PHARMACEUTIQUES MIXTES DESTINEES AU TRAITEMENT DE MALADIES RHUMATISMALES

(84) Benannte Vertragsstaaten:
**AT BE CH DE DK ES FI FR GB GR IE IT LI LU NL PT SE**

(30) Priorität: **08.08.1997 DE 19734293**

(43) Veröffentlichungstag der Anmeldung:
**27.09.2000 Patentblatt 2000/39**

(73) Patentinhaber: **Roche Diagnostics GmbH
68298 Mannheim (DE)**

(72) Erfinder:
• **LEHMANN, Paul
D-67549 Worms (DE)**
• **KALTWASSER, Joachim, Peter
D-60389 Frankfurt am Main (DE)**

(74) Vertreter: **Notegen, Eric-André, Dr. et al
Grenzacherstrasse 124
4070 Basel (CH)**

(56) Entgegenhaltungen:
EP-A- 0 025 721          WO-A-96/14081
WO-A-97/09996          GB-A- 2 171 304

• NORDSTROM D ET AL: "Availability of iron and degree of inflammation modifies the response to recombinant human erythropoietin when treating anemia of chronic disease in patients with rheumatoid arthritis." RHEUMATOLOGY INTERNATIONAL, (1997) 17 (2) 67-73. JOURNAL CODE: TDZ. ISSN: 0172-8172., XP002093019 GERMANY: Germany, Federal Republic of
• HOCHLI P ET AL: "Repeated continuous administration of low doses of intravenous iron in anemic patients with active rheumatoid arthritis [letter]." EUROPEAN JOURNAL OF HAEMATOLOGY, (1993 JUL) 51 (1) 54-5. JOURNAL CODE: ERF. ISSN: 0902-4441., XP002093020 Denmark

**Beschreibung**

Die vorliegende Erfindung betrifft die Verwendung von Erythropoietin und Eisenpräparaten zur Herstellung von pharmazeutischen Kombinationspräparaten. Diese Kombinationspräparate umfassen einzelne Darreichungsformen eines Erythropoietin-Präparates und ein physiologisch verträgliches Eisenpräparat entsprechend einer äquivalenten Menge von 1 - 40 mg an Eisenionen zur Behandlung von rheumatischen Erkrankungen.

Pharmazeutische Kombinationspräparate enthaltend Erythropoietin- und Eisenpräparate sind aus der PCT-Patentanmeldung WO 97/09996 bekannt. Die Präparate werden insbesondere zur Optimierung der Erythropoese bei der Behandlung von Krankheiten eingesetzt, bei denen eine Stimulierung der Erythrozytenbildung angestrebt wird.

Aus WO 96/14081 ist die Verwendung von Erythropoietin zur Behandlung von chronischen Entzündungen, insbesondere der rheumatoiden Arthritis, bekannt.

In GB-A-2,171,304 und in Rheumatol. Int., 1997, 17:67-73 (Nordström et al.) wird die Verwendung von EPO zur Behandlung von Anämie, speziell von Anämie bei rheumatoider Arthritis, ggf. in Kombination mit hohen Eisenmengen i.v. vorgeschlagen. Es hat sich jedoch gezeigt, dass i.v. Eisen, in hohen Mengen appliziert, toxische Wirkungen zeigt.

Die therapeutische Behandlung von Patienten, die an rheumatischen Erkrankungen leiden, ist bis heute immer noch nicht mit einem zufriedenstellenden Behandlungserfolg möglich. Insofern besteht ein Bedürfnis nach besseren Behandlungsmethoden, wie beispielsweise bei der Behandlung der rheumatoiden Arthritis, Lupus erythematodes, Morbus Bechterew etc.

Rheumatische Erkrankungen des Bewegungsapparates und entzündliche Gelenkerkrankungen sind weltweit eine der Hauptursachen für chronische Schmerzen und schwere körperliche Beeinträchtigung. Alle Elemente des muskuloskeletalen Apparates befinden sich in einem dynamischen Gleichgewicht und verändern - in Abhängigkeit von Belastung und mechanischen Erfordernissen - ständig ihre Form, Struktur und ihren Funktionszustand. Dieses System ist anfällig für Traumata und empfänglich für lokalisierte und systemische entzündliche Erkrankungen. Akute entzündliche Zustände oder Gewebeschädigungen resultieren oft in chronischen Zuständen, möglicherweise wegen der ständigen Bewegungen und mechanischen Belastungen.

Gelenkerkrankungen gehören zu den Erkrankungen des Bewegungsapparates und werden in solche, die das periartikuläre Gewebe betreffen und echte Gelenkerkrankungen (z.B. Arthrose) unterteilt. Symptome und Befunde lassen sich oft auf eine systemische, generalisierte Erkrankung oder auch auf Krankheiten, die primär von einem anderen System oder Organ ausgehen, zurückführen.

Im pathophysiologischen Ablauf rheumatischer Krankheiten spielt die immunvermittelte Entzündung eine wichtige Rolle. In vielen Fällen ist die immunvermittelte Entzündung die Basis zahlreicher systemischer Bindegewebserkrankungen. Auch infektiöse Prozesse sind von Bedeutung, vor allem bei Krankheiten wie rheumatischem Fieber, der Lyme-Borreliose oder der reaktiven Arthritis. Die Ätiologie rheumatischer Erkrankungen dürfte multifalctoriell sein, wobei auch genetische Ursachen und Umgebungseinflüsse einen wichtigen Einfluß haben.

Schmerz ist ein Hauptsymptom der meisten rheumatischen Erkrankungen, insbesondere bei Gelenkerkrankungen. Bis heute sind die Ursachen des Gelenkschmerzes weitgehend ungeklärt. Eine therapeutische Kontrolle des Gelenkschmerzes ist derzeit nur unzureichend möglich. Auch die ausschlaggebenden Faktoren eines Funktionsverlustes sind erst unvollständig geklärt. Viele der wichtigsten Gelenkerkrankungen zeigen in ihrer Inzidenz bemerkenswerte Geschlechtsunterschiede; so tritt der SLE vor allem bei Frauen, die Spondylitis ankylosans häufiger und in schwererer Form bei Männern auf Die Gründe hierfür sind ebenfalls unklar.

Inzidenz. Ausprägung und Auswirkungen der Krankheiten des Bewegungsapparates sind abhängig vom Alter und Geschlecht. Einige Krankheiten treten nur im Kindesalter auf (Juvenile chronische Arthritis); andere, wie SLE oder Spondylitis ankylosans, beginnen meist bei jungen Erwachsenen. Polymyalgia rheumatica und Riesenzellarterütis treten dagegen fast nie vor dem 55. Lebensjahr auf cP, SLE, Gicht und andere wichtige entzündliche rheumatische Erkrankungen zeigen bei Beginn im höheren Alter eine andere Ausprägung. Die meisten dieser Erkrankungen des Bewegungsapparates verursachen chronischen Schmerz.

Für die Heilung dieser chronischen rheumatischen Erkrankungen gibt es keine zufriedenstellend wirkenden Arzneimittel. Die bei der Behandlung verwendeten therapeutischen Prinzipien ähneln sich häufig und hängen oft von Faktoren wie Alter und Gesamtsituation des Patienten, Ausmaß der entzündlichen Aktivität und von den Folgen (Schmerzintensität, Ausmaß der Behinderung) ab. Der in der Praxis verwendete Behandlungsplan für Patienten mit schweren Krankheiten des Bewegungsapparates setzt sich aus verschiedenen Maßnahmen zusammen, und ist insofern oft abhängig vom behandelnden Arzt. Eine einheitliche und allgemein akzeptierte Behandlungsmethode hat sich bislang nicht etabliert.

Überraschenderweise wurde nun gefunden, daß mit Hilfe einer optimalen Menge an EPO und Eisen in Form eines entsprechenden Kombinationspräparates eine positive Beeinflussung des gesamten Krankheitsbildes bei Patienten mit rheumatischen Erkrankungen und eine Besserung des Allgemeinbefindens und der Lebensqualität dieser Patienten erzielt werden kann. Ferner wird eine optimale Wirkung von EPO bzw. der eingesetzten Eisenpräparate erreicht. Insbesondere können hierdurch die Kosten für die Behandlung mit EPO deutlich reduziert werden, indem beispielsweise geringere Dosierungen des Wirkstoffes verabreicht werden können. Die erfindungsgemäßen Kombinationspräparate eignen sich insbesondere zur Behandlung von entzündlichen Gelenkerkrankungen. Ferner kann bei vielen Patienten eine deutliche Schmerzlinderung erreicht werden.

Im Sinne der vorliegenden Erfindung werden insbesondere solche pharmazeutische Kombinationspräparate verwendet, die 250 - 20.000 U eines Erythropoietin-Präparates und 1 - 40 mg einer äquivalenten Menge an Eisenionen eines physiologisch verträglichen Eisenpräparates umfassen, wobei das Eryhtropoietin-Präparat und das Eisenpräparat in getrennten Darreichungsformen oder in einer einheitlichen Darreichungsform vorliegen können. Die Kombinationspräparate enthalten vorzugsweise 1 - 30 mg, besonders bevorzugt 3 - 20 mg, einer äquivalenten Menge an Eisenionen eines physiologisch verträglichen Eisenpräparates, insbesondere eines Fe(II)- oder Fe(III)-komplexes.

Als geeignete Erythropoietin-Präparate im Sinne der vorliegenden Erfindung kommen solche Wirkstoffe in Frage, die hinsichtlich der physiologischen Wirkung des humanen EPOs vergleichbar sind. Geeignete EPO-Präparate sind beispielsweise das rekombinante humane EPO (rhEPO; vgl. Europäische Patentschrift EP 0,205,564 bzw. EP 0,411,678) oder auch entsprechende Modifikationen derartiger Proteine. Als Modifikationen kommen beispielsweise solche Proteine mit höherem oder geringerem Molekulargewicht als 34.000 Da (Molekulargewicht des urinären EPO) in Frage, ebenso Isoformen des Enzyms oder Proteine mit unterschiedlicher Glykosylierung. Insbesondere können auch durch PEG (Polyethylenglykol) chemisch modifizierte Proteine verwendet werden. Ferner kommen grundsätzlich auch solche Proteine in Frage, die sich durch Deletionen. Substitutionen oder Verlängerungen von einzelnen oder mehreren Aminosäuren von der Aminosäuresequenz des natürlichen EPO mit einer Länge von 166 Aminosäuren ableiten. Derartige Proteine besitzen im wesentlichen vergleichbare physiologische Eigenschaften wie rhEPO. Insbesondere weisen derartige Proteine biologische Eigenschaften auf, daß Knochenmarkszellen veranlasst werden, die Produktion von Retikulozyten und roten Blutkörperchen zu steigern und/oder die Hämoglobinsynthese oder Eisenaufnahme zu steigern. Anstelle derartiger Proteine können auch niedermolekulare Substanzen verwendet werden, die als EPO-Mimetika bezeichnet werden, und die an den gleichen biologischen Kezeptor binden. Diese Mimetika können vorzugsweise auch oral verabreicht werden. Die zu verabreichende Menge derartiger Proteine oder Mimetika wird ermittelt durch Vergleich der biologischen Aktivitäten zwischen EPO und diesen Wirkstoffen.

Die orale Eisenresorption beträgt nur ca. 1 mg pro Tag, unter extremer Belastung (bei oraler Verabreichung von ca. 300 mg Fe(III)/Tag) weniger als 3 mg pro Tag. Daher wird zunehmend die intravenöse Applikation von Eisenpräparaten bevorzugt. Auf dem deutschen Arzneimittelmarkt stehen momentan zwei intravenös applizierbare Eisen-Präparate zur Verfügung. Dabei handelt es sich um die Medikamente "Ferrlecit" und "Ferrum Vitis". Ferrlecit ist ein Eisen-(3)-Gluconat-Komplex, während "Ferrum Vitis" ein Eisen-(3)-Hydroxid-Saccharat-Komplex ist.

Die vielfältigen Probleme einer hochdosierten, langfristigen oralen Eisen-Therapie lassen sich zwar relativ einfach durch die intravenöse, subcutane Applikation von physiologisch verträglichen Eisen(III)-Salzen während der Hämodialysebehandlung umgehen, da hierbei ein sicherer intravenöser, subcutaner Zugang besteht und die Injektion ohne weitere Belastung für den Patienten erfolgen kann. Die intravenöse Verabreichung von Eisen-Präparate ist jedoch nicht triviaL da bei der Applikation von hohen Dosen mit Nebenwirkungen zu rechnen ist, vor allem dann, wenn größere Mengen relativ schnell injiziert werden müssen. Ferner kann die intravenöse Verabreichung der Eisenpräparate Probleme bereiten bis hin zu Akute-Phasen-Reaktionen, wenn die Eisendosis zu hoch bzw. nicht optimal abgestimmt mit der EPO-Dosis gegeben wird. Bei einer zu hohen Dosierung von eisenhaltigen Präparaten kann es auch zu Eisenvergiftungen kommen. Elementares Eisen hat einen toxischen Effekt auf den Gastrointestinaltrakt. das kardiovaskuläre und des zentralnervöse System. Die oral letale Dosis elementaren Eisens schwankt zwischen 200 und 250 mg/kg. Die am häufigsten verwendeten Eisentabletten sind Ferrosulfat (enthält ca. 20 % elementares Eisen), Ferrofumarat (enthält etwa 30 % elementares Eisen) oder Ferroglukonat (enthält ca. 10 % elementares Eisen).

Eisenpräparate im Sinne der vorliegenden Erfindung sind orale oder parenterale Darreichungsformen. Hierbei kann es sich grundsätzlich um Einzelpräparate handeln, die als Wirkstoff ein physiologisch verträgliches Eisensalz oder eine Eisenkomplex-Verbindung enthalten, oder auch um Kombinationspräparate, die neben dem physiologisch verträglichen Eisenpräparat weitere Wirkstoffe, wie z.B. Vitamine, Folsäure, Thiaminchlorid, Riboflavin, Pyridoxin, Ascorbinsäure, Nicotinamid, Calciumpantothenat, etc., enthalten Physiologisch verträgliche Eisensalze oder Eisenkomplex-Verbindungen sind beispielsweise Eisen(II)-sulfat, Eisen(II)-fumarat, Eisen(III)-citrat, Eisen(II)-gluconat, Eisen(II)-succinat. Eisen(II)-chlorid, Eisen(II)-glycin-sulfat-Komplex, Eisen(II)-aspartat, Natrium-Eisen(III)-gluconat-Komplex, Eisen(III)-hydroxid-Polymaltose-Komplex oder Ferri-Sorbitol-Zitrat-Komplex. Bevorzugte Eisen-präparate sind insbesondere Fe(III)-Komplexe, insbesondere solche mit einem Molekulargewicht zwischen 30.000 und 100.000 D. Besonders bevorzugt ist Fe(III)-Saccharat. Hier kann auf das kommerziell erhältliche Präparat "Ferrum Vitis" (Fa. Neopharma, Deutschland) zurückgegriffen werden. Durch die erfindungsgemäße geringe Eisendosierung ist es auch möglich, labile Eisen-Komplexe, wie das Eisen-Gluconat (MG ca. 1000 D; Ferrlecit), im Kombinationspräparat einzusetzen, obwohl diese labilen Eisenkomplexe relativ große Mengen ionisiertes Eisen freisetzen, was bei der intravenösen Applikation größerer Mengen zu Toxizitäten führen würde.

Im folgenden wird bei Bezugnahme auf die Menge des Eisenpräparates grundsätzlich die zu applizierende äquivalente Menge an Eisenionen, Fe(II)- oder Fe(III)-ionen, verstanden. Durch diese Standardisierung kann die Menge eines beliebigen Eisenpräparates auf Basis dessen bekannten Molekulargewichts berechnet werden. Im Fall des Eisen-(III)-gluconat x 2 $H_2O$ beträgt beispielsweise die Menge an Eisen 80,5 mg, wenn eine Menge von 695 mg des Eisenpräparates verabreicht wird. Bei Verabreichung von beispielsweise 280 mg wasserfreiem Eisen(II)-succinat beträgt die Eisenmenge 95.2 mg.

Im Sinne der vorliegenden Erfindung sollen unter dem Begriff "Kombinationspräparate" nicht nur solche Arzneimittelpackungen verstanden werden, bei denen das EPO-Präparat und das Eisenpräparat in einer verkaufsfertigen Verpackungseinheit nebeneinander konfektioniert vorliegen (sogenannte Kombinationspackung), sondern auch solche Arzneimittelpackungen, die entweder eine geeignete Menge eines EPO-Präparates oder eine geeignete Menge eines Eisenpräparates in Form der jeweiligen Einzelpräparate enthalten, wobei die Einzelpräparate hinsichtlich der Menge der Inhaltsstoffe derart konfektioniert sind. daß sie im Sinne der Erfindung für die kombinierte Gabe mit dem jeweils anderen Präparat verabreicht werden können. In diesen Fällen werden den Präparaten in der Regel von den Pharma-Herstellern oder den Arzneimittel-Importeuren ein in vielen Ländern gesetzlich vorgeschriebener Beipackzettel für Arzneimittel beigelegt, in dem Anweisungen oder Informationen über die kombinierte Gabe der Einzelpräparate enthalten sind. Die Kombinationspräparate können vorzugsweise in einer einheitlichen Darreichungsform vorliegen, in der die jeweilige Menge des EPO-Präparates und des Eisenpräparates nebeneinander in einem Behältnis vorliegen.

Für die Behandlung von Hämodialysepatienten beinhaltet das erfindungsgemäße Kombinationspräparat beispielsweise 250 bis 15.000 U (anstelle der Abkürzung "U" kann auch die Abkürzung "IE" für Internationale Einheiten verwendet werden) eines EPO-Präparates, insbesondere 500 bis 10.000 U. Bevorzugte Dosierungen sind 250 U, 500 U, 1.000 U, 2.000 U, 5.000 U, 7.500 U und 10.000 U pro Einzeldosierung. Die Menge an Eisenionen beträgt vorteilhaft bis zu 30 mg, insbesondere 3 - 20 mg, vorzugsweise 5 - 20 mg und besonders bevorzugt etwa 10 mg. Für die Behandlung von Anämiepatienten beträgt die optimale Dosis 500 bis 10.000 U, vorzugsweise etwa 1.000 - 3.000 U. Die Menge an Eisenionen beträgt in diesem Fall vorteilhaft bis zu 30 mg, beispielsweise 3 - 15 mg und insbesondere etwa 5 mg.

Die erfindungsgemäßen Konzentrationen des EPO-Präparates und des Eisenkomplexes erlauben in ihrer Kombination eine optimale Einstellung und Behandlung von Patienten, die unter rheumatischen Erkrankungen leiden, und führen bei intravenöser Eisentherapie nicht zu Akute-Phase-Reaktionen.

Die Behandlung mit dem Kombinationspräparat erfolgt ein- bis fünfmal, bevorzugt bis zu viermal wöchentlich, wobei die Gesamtmenge an Eisenionen pro Patient von 100 mg pro Woche nicht überschritten wird. Bei der Behandlung von Patienten mit rheumatischen Erkrankungen sollte vorteilhaft eine Gesamtmenge von 80 mg, insbesondere 50 mg an Eisenionen pro Woche nicht überschritten werden. Ein besonderer Vorteil des erfindungsgemäßen Kombinationspräparates in der klinischen Praxis liegt darin, daß es sowohl in der Korrektur- als auch in der Erhaltungsphase der Eisentherapie bei Hämodialysepatienten angewendet werden kann, ohne Toxizitäten hervorzurufen. Bisher wurden unterschiedliche Mengen an Eisen verabreicht, wobei in der Korrekturphase zunächst höhere Dosierungen an Eisenionen im Vergleich zur Erhaltungsphase verabreicht wurden. Überraschenderweise ist diese unterschiedliche Dosierung bei Verwendung der erfindungsgemäßen Kombinationspräparaten nicht mehr erforderlich. Die Menge des Erythropoietin-Präparates und des Eisenpräparates sind bei den erfindungsgemäßen Kombinationspräparates derart optimal aufeinander abgestimmt, daß eine Unterscheidung zwischen Erhaltungsdosis und Korrekturdosis nicht erforderlich ist. Hierdurch wird eine erhöhte Sicherheit bei der Behandlung der Patienten erzielt, da Verwechslungsmöglichkeiten bezüglich der optimalen Dosierung des Eisenpräparates nicht mehr bestehen.

In einer weiteren bevorzugten Ausführungsvariante enthält das Kombinationspräparat als weitere Komponente ein Präparat, das eine suppressive Wirkung auf TNF-alpha, besitzt. Vorzugsweise handelt es sich um Gluco-Corticosteroide, wie z.B. Cortison, Cortisol-Analoga oder Prednisolon-Derivate oder um Antimetaboliten der Folsäure, wie z. B. Methotrexate. Bevorzugte Verbindungen sind Prednison, Prednilolon, $6\alpha$-Methylprednisolon, Triamcinolon, Paramethason, Dexamethason, Betmethason, Cortison, Cortisol und 16-Methylen-prednisolon. Die antientzündliche Wirkung der EPO/Eisengabe wird synergistisch verstärkt.

Bei der Anwendung der Kombinationspräparate ist es möglich, die Präparate, vorzugsweise das EPO-Präparat und das Eisenpräparat in einer sogenannten fixen Kombination, d.h. in einer einzigen pharmazeutischen Formulierung zu verabreichen. in der die Verbindungen enthalten sind. Dies können z.B. Injektionslösungen. Infusionslösungen oder Lyophilisate sein, die beispielsweise in Ampullen abgefüllt sind. Diese Darreichungsform hat den Vorteil, daß das EPO-Präparat bei der Herstellung und Lagerung der Darreichungsform durch den Eisenkomplex stabilisiert wird. Die fixe Kombination der Wirkstoffe in Form eines Lyophilisates hat den weiteren Vorteil der einfachen und sicheren Handhabung Das Lyophilisat wird in der Ampulle durch Zugabe pharmazeutisch üblicher Injektionsmedien gelöst und intravenös appliziert.

Es ist auch möglich, die Präparate in Form von getrennten pharmazeutischen Formulierungen zur Verfügung zu stellen. In der Regel erfolgt dies in Form einer einzigen Verpackungseinheit, die mehrere Behältnisse umfaßt, wobei das erste Behältnis eine das Erythropoietin-Präparat enthaltende Darreichungsform (Lyophilisat, Injektions- oder Infusionslösung) ist, und das zweite Behältnis eine geeignete Darreichungsform für das Eisenpräparat und ggf. das dritte Behältnis eine geeignete Darreichungsform eines TNF-alpha-Suppressors darstellt. Die Verpackungseinheiten können auch mehrere Einzeldosierungspräparate der jeweiligen Präparate enthalten, so daß beispielsweise eine Verpackungseinheit die für einen bestimmten Zeitraum (z.B. für die wöchentliche Dosierung) erforderliche Anzahl von Einzeldarreichungsformen beinhaltet.

Diese freie Kombination, die in einer einzigen Verpackungseinheit (Arzneimittelpackung) zur Verfügung gestellt werden kann, hat den Vorteil, daß jedem zu behandelnden Patienten eine bestimmte individuelle Menge eines EPO-Präparates, eines Eisenpräparates und ggf. eines TNF-alpha-Suppressors zugeordnet werden kann. Derartige Kombinationspräparate bieten außerdem den Vorteil der größeren Sicherheit für den Therapieerfolg, da jeweils die optimal abgestimmte Menge der Einzelpräparate festgelegt ist, und eine Verwechslung mit sonst im Handel erhältlichen Einzelpräparten, die in unterschiedlichen Dosierungen angeboten werden, weitgehend ausgeschlossen werden kann. Zudem ist zu berücksichtigen, daß in verschiedenen Ländern oft Arzneimittelpräparate mit unterschiedlichen Dosierungen aufgrund der nationalen Erfordernisse im Handel sind, und somit eine erhöhte Verwechslungsgefahr mit variierenden Mengenverhältnisse der Einzelwirkstoffe besteht. Die erfindungsgemäßen Kombinationspräparate minimieren ferner das Risiko einer versehentlich zu hohen Eisengabe, die möglicherweise erfolgen kann, wenn herkömmliche Eisenpräparate aus separaten Arzneimittelpackungen zusammen mit der Gabe eines Erythropoietin-Präparates eingesetzt werden. Durch die erfindungsgemäßen Kombinationspräparate wird eine sichere Therpie und einfache Handhabung durch das behandelnde Personal oder im Rahmen der durch den Patienten vorgenommenen Selbstmedikation sichergestellt. Im vorliegenden Fall ist es z.B. auch möglich, einen Wirkstoff als Injektionslösung und den anderen Wirkstoff (Eisenkomplex) als Darreichungsform zur oralen Verabreichung zur Verfügung zu stellen.

Für den Fall, daß das EPO-Präparat als Lyophilisat zur Verfügung gestellt wird, enthalten die Arzneimittelpackungen (Kombinationspackungen) die entsprechende Menge des EPO-Präparates in Glasampullen oder in Karpulen. Das Eisenpräparat kann in fester Form (Tablette, Pulver, Granulat, Lyophilisat, etc.) oder auch in flüssiger Form in einem getrennten Behältnis vorliegen. Ferner enthält die Kombinationspackung vorzugsweise eine Rekonstitutionslösung, um entweder das Wirkstofflyophilisat allein oder auch zusammen mit dem festen Eisenpräparat aufzulösen. Liegt das Eisenpräparat als gebrauchsfertige Lösung vor, kann die Lösung zusammen mit der EPO-Lösung gemischt werden, falls die gemeinsame Applikation von EPO und Eisenpräparat erfolgen soll. Grundsätzlich kann das Eisenpräparat auch als Konzentrat für den Zusatz zu herkömmlichen Infusionslösungen zur Verfügung gestellt werden, wodurch eine langsamere Applikation über mehrere Stunden hinweg erfolgen kann. In diesem Fall wird ein geringes Volumen der Eisenkomplex-haltigen Lösung (ca. 0,5 - 10 ml) zur gebrauchsfertigen Injektionslösung von ca. 500 - 1000 ml hinzugefügt.

Kombinationspräparate im Sinne der vorliegenden Erfindung sind auch solche Verpackungseinheiten, die auf eine optimale wöchentlich zu verabreichende Menge des EPO-Präparates und des Eisenpräparates abgestellt sind. Vorteilhaft werden wöchentlich 5.000 - 50.000 U eines EPO-Präparates verabreicht. Diese Gesamtdosis kann in mehreren Teildosierungen für die tägliche Gabe (d.h. 7 mal pro Woche) oder für die Verabreichung von 1 - 6 Teilmengen pro Woche aufgeteilt sein. Die wöchentlich zu verabreichende Menge des Eisenpräparates kann gegebenenfalls in einer der wöchentlichen Gesamtdosis entsprechenden Menge oder auch in mehreren Teilmengen für eine mehrmalige Gabe pro Woche zusammen mit dem Erythropoietin-Präparat aufgeteilt sein.

Eine weitere Möglichkeit im Sinne der vorliegenden Erfindung besteht darin, jeweils einzelne Darreichungsformen des Erythropoietin-Präparates oder des Eisenpräparates als unabhängige Arzneimittel zur Verfügung zu stellen, wobei die Einzelpräparate derart konfektioniert sind, daß sie die erforderlichen Menge der Einzelsubstanzen für die erfindungsgemäße Kombination des EPO-Präparates und des Eisenkomplexes enthalten. In der Regel enthalten die Arzneimittelpackungen die vorgeschriebenen Beipackzettel, in denen ein entsprechender Hinweis für die kombinierte Gabe mit EPO bzw. mit Eisenpräparaten in der erforderlichen Menge enthalten ist. Ein entsprechender Hinweis kann auch als Verpackungsaufdruck auf der Arzneimittelpackung (Sekundärpackmittel) oder dem Primärpackmittel (Ampulle, Blisterstreifen, etc.) enthalten sein. So wird im Falle des EPO-haltigen Arzneimittels mit 250 - 20.000 Units EPO beispielsweise darauf hingewiesen, daß dieses Präparat insbesondere zusammen mit einem Eisenkomplex-Präparat enthaltend 1 - 40 mg , vorzugsweise 5 - 30 mg Eisen verabreicht werden sollte. Im Falle der Eisenpräparate wird umgekehrt auf die kombinierte Gabe mit 250 - 20.000 U eines Erythropoietin-Präparates hingewiesen.

Eine weitere Möglichkeit der Bereitstellung der EPO-Präparate besteht darin, entsprechende Multi-Dose-Präparate zur Verfügung zu stellen, die das EPO-Präparat in höheren Mengen im Vergleich zur Einzeldosierung enthalten. Derartige Präparate sind insbesondere für den Einsatz in Kliniken geeignet, bei denen täglich eine Vielzahl von Patienten behandelt wird. Diese Multi-Dose-Präparate enthalten die EPO-Präparate in Dosierungen von bis zu 500.000 U, insbesondere bis zu 100.000 U oder 50.000 U. Die Multi-Dose-Präparate haben den Vorteil, daß sie eine beliebige Dosierungsentnahme des EPO-Präparates durch das medizinische Fachpersonal ermöglichen, beispielsweise durch Entnahme von entsprechenden Volumenanteilen der injektionsfertigen Lösung. Dies ist insbesondere vorteilhaft bei der Behandlung von Patienten mit unterschiedlichem Dosierungsbedarf des Wirkstoffes oder bei der Behandlung von Kindern, die denen eine geringere Dosierung des EPO-Präparates erforderlich ist. Aus einer vorzugsweise zu Beginn des Tages frisch hergestellten Injektionslösung von beispielsweise 100.000 U eines EPO-Präparates können unter Umständen alle während dieses Tages anfallenden Behandlungen der Patienten durchgeführt werden, ohne daß die Herstellung von getrennten Injektionslösungen für jeden einzelnen Patienten erforderlich ist. Dies kann zu einer signifikanten Zeitersparnis bzw. zu einer Arbeitsentlastung des medizinischen Fachpersonals führen. Vorzugsweise werden die einzelne EPO-Dosierungen im Bereich von 250 U, 500 U, 1000 U und 10.000 U entnommen.

Die Multi-Dose-Präparate können auch in Form von Lösungen vorliegen, die in Karpulen abgefüllt sind. Diese Karpulen eignen sich zum Einsatz in sogenannten Pens, die eine Verabreichung durch den Patienten selbst und eine individuelle Dosierungsentnahme ermöglichen. Beispielsweise enthalten derartige Karpulen das EPO-Präparat in einer Menge von 10.000 oder 20.000 U, wobei durch entsprechende Einstellung des Entnahmevolumens Dosierungsintervalle von beispielsweise 250 U, 500 U, 1.000 U oder 2.000 U möglich sind.

Die Herstellung der pharmazeutischen Darreichungsformen erfolgt nach üblichen, in der galenischen Technik bekannten Verfahren mit pharmazeutisch üblichen Hilfsstoffen.

Im Zusammenhang mit der Diagnose von rheumatischen Erkrankungen und von Eisenstoffwechselstörungen kann im Sinne der vorliegenden Erfindung insbesondere die Serumferritin-Konzentration bestimmt werden. Tritt zusätzlich zu einer bereits vorhandenen rheumatischen Erkrankung oder Anämie ein echter Eisenmangel auf, so steigt das Ferritin nicht an (meist bleibt es unter 90-95 ng/ml). Dieser Wert weist bei gleichzeitigen klinischen Zeichen von Infektion, Entzündung oder maligner Erkrankung auf eine Kombination von Eisenmangel und Anämie in Begleitung mit einer rheumatischen Erkrankung hin. Da das Serumferritin bei diesen Erkrankungen auch im Sinne eines Akute-Phase-Proteins reagieren kann, kann das Erythrozytenferritin besser diagnostisch verwertet werden. Das bei der Erythropoese nicht benötigte Eisen wird mittels Transferrin in zwei Arten des Speicherpools verlagert. Der wichtigste Speicher ist das Ferritin. Hierbei handelt es sich um eine heterogene Familie von Proteinen, die einen Eisenkern umschließen. Es ist löslich und stellt die aktive Speicherform in Leber (Hepatozyten), Knochenmark, Milz (Makrophagen), Erythrozyten und im Serum (ca. 100-300 µg/l) dar. Der Gewebeferritinpool ist sehr labil und rasch verfügbar, wenn Eisen benötigt wird. Das zirkulierende Serum-Ferritin stammt aus dem retikuloendothelialen System, und seine zirkulierende Konzentration geht parallel mit dem Gesamtkörpereisen (jedes ng/ml entspricht 8 mg Eisenvorrat).

Bei der Durchführung der Kombinationstherapie mit dem erfindungsgemäßen Kombinationspräparat kann auf sehr einfache Art und Weise über die wöchentliche maximale Dosierung entschieden werden, indem die diagnostischen Parameter für den Eisenstatus, insbesondere die Parameter Eisen, Transferrin, Transferrinsättigung, Transferrinrezeptor und Ferritin, bestimmt werden. Es zeigte sich, daß der Patient in Korrektur- und Erhaltungsphase optimal eingestellt ist, wenn

| Ferritin | 100 - 300 µg/l (entspricht Speicher-Eisen(III) von 800 - 1200 mg), und die |
| Transferrinsättigung | 20 - 40 % |

betragen. Bevorzugt beträgt die Ferritin-Konzentration mindestens 100 µg/l, insbesondere mindestens 150 µg/l, und maximal bis zu 300 µg/l, insbesondere maximal bis zu 250 µg/l. Die Eisenkonzentration beträgt vorteilhaft zwischen 10 - 20 µmol/l (entspricht etwa 56 - 112 µg/dl) und die Transferrinkonzentration zwischen 30 - 60 µmol/l (entspricht etwa 240 - 480 mg/dl). Die Transferrinsättigung ist definiert als das Verhältnis von Serum/Plasma-Eisenkonzentration zu Serum/Plasma-Transferrin-Konzentration (multipliziert mit einem Korrekturfaktor von 1,41). Es handelt sich hierbei um eine dimensionslose Zahl, die unabhängig von Hydratationsstatus des Patienten ist. Die Transferrinsättigung berechnet sich nach der Formel:

$$\text{Transferrinsättigung (\%)} = (\text{Eisen [mg/dl]} \times 100) / (\text{Transferrin [mg/dl]} \times 1{,}41)$$

Eine optimale Einstellung des Patienten ist erreicht, wenn das Verhältnis von Transferrinsättigung (in %) zur Ferritinkonzentration (in µg/l) im Bereich von 5 - 40 % liegt. Dieser Parameter wird definiert als Transferrin/Ferritin-Sättigung (TfF-Sättigung). Er berechnet sich nach der Formel

$$\text{TfF-Sättigung} = (\text{Transferin-Sättigung in \%}) \times 100 / (\text{Ferritin [µg/l]}.)$$

Bevorzugt liegt der Wert für diesen Parameter im Bereich von 10 - 40, insbesondere bei 15 - 25 [% x 1 / µg].

Mittels dieser Parameter wird z.B. bei Verabreichung von 1 bis 6 Ampullen, vorzugsweise bis zu 3, 4 oder 5 Ampullen, in der Woche (eine Ampulle enthält 500 - 7.500 U rhEPO und 1 - 20 mg Eisenkomplex) die optimale Einstellung des Patienten diagnostisch überprüft.

Um unerwünschte Nebenwirkungen sicher auszuschließen, wird der Akute-Phase-Parameter CRP (5 mg/l ± 100%) [CRP = C-reaktives Protein] gemessen, wobei das CRP zur Zeit als der beste Protein-Marker einer Entzündungsreaktion gilt. Weitere Parameter sind TNF-alpha (Tumor Necrosis Faktor alpha) und IL-6 (Interleukin 6) bzw. IL-1, IL-2 und IL-8. TNF-alpha sollte < 30 pg/ml (im Plasma, ELISA) und IL-6 < 20 pg/ml (im Plasma, ELISA) betragen. Zusätzlich können die Leberparameter GPT (Glutamat-Pyruvat-Transaminase), GOT (Glutamat-Oxalacetat-Transaminase) und γ-GT (Gamma-Glutamyltransferase) bestimmt werden, die in folgenden -Bereichen liegen sollten (Bestimmung bei 37°C): GPT: < 50 U/l; GOT: < 50 U/l; γ-GT: < 40 U/l. Der Parameter GPT steht hierbei gegenwärtig in der Leberdiagnostik an erster Stelle.

Desweiteren können gegebenenfalls die hämatologischen Kontrollparameter wie Hämatokrit (Anteil der roten Blutkörperchen am Gesamtvolumen) oder der Anstieg der hypochromen Erythrocyten herangezogen werden. Zeigen die Kontrollparameter höhere Anstiege, ist die wöchentliche Eisen-Gabe zu reduzieren, dann sollte zusätzlich rhEPO verabreicht werden. Zeigen die Kontrollparameter, vor allem die Transferrinsättigung, geringere Werte, ist die wöchentliche Eisen-Gabe zu erhöhen.

Wie bereits ausgeführt, wird, um unerwünschte Nebenwirkungen sicher auszuschließen, der Akute-Phase-Parameter CRP (2 - 10 mg/l ) [CRP = C-reaktives Protein] gemessen, zusätzlich kann der Leberparameter Leberparameter GPT (Glutamat-Pyruvat-Transaminase), bestimmt werden, der < 50 U/l bei 37°C (< 30 U/l bei 25 °C) sein sollte. Desweiteren können gegebenenfalls die hämatologischen Kontrollparameter wie Hämatokrit (Anteil der roten Blutkörperchen am Gesamtvolumen) oder der Anstieg der hypochromen Erythrocyten herangezogen werden. Hierbei können die Retikulozyten auf einen Wert von bis zu 15/1000 - 30/1000 steigen. Die typtische Hämoglobin-Konzentration liegt bei 12 -18 g/dl. Zeigt der lösliche TfR einen höheren Anstieg, ist die wöchentliche Eisen-Gabe zu erhöhen auf bis zu 35 mg. Zeigt der lösliche TfR geringere Werte, ist die wöchentliche EPO-Dosis zu erhöhen.

Die Bestimmung des Eisenstatus erfolgt durch Analyse von Proben aus Körperflüssigkeiten (Blut, Serum, Urin etc.) der betreffenden Patienten. Für die Bestimmung des Eisenstatus werden insbesondere die Konzentration von Eisen, Transferrin, Ferritin, Transferrinrezeptor, die Transferrinsättigung und die Transferrin/Ferritin-Sättigung bestimmt. Im Falle von Hämodialysepatienten werden vorzugsweise die Parameter Eisen, Transferrin, Ferritin und Transferrinsättigung nach an sich üblichen Analysenmethoden bestimmt. Relevant ist insbesondere die Bestimmung des Transferrin/Ferritin-Sättigungswertes. Im Falle von Anämie-Patienten, deren Anämie nicht durch Hämodialyse verursacht ist, werden vor allem die Ferritinkonzentration und die Konzentration des Transferrin-Rezeptors bestimmt. Relevant ist insbesondere die Bestimmung des Verhältnisses von Transferrin-Rezeptor zu Ferritin (Transferrin-Rezeptor/Ferritin-Sättigungswert).

Ein in diesem Sinne erfindungsgemäßes optimales Kombinationspräparat für die Behandlung von Patienten mit rheumatischen Erkrankungen umfaßt 500 - 10.000 U. insbesondere 2.000 - 4.000 U eines EPO-Präparates und 3 - 10 mg, vozugsweise 5 mg an Eisenionen, vorzugsweise eines Fe(III)-Komplexes, wobei das EPO-Präparat und der Fe(III)-komplex in getrennten Darreichungsformen oder in einer einheitlichen Darreichungsform vorliegen können. Die erfindungsgemäßen Darreichungsformen ermöglichen auch eine Applikation der Eisenpräparate 1 bis 3 Tage vor der EPO-Applikation, um die Eisenspeicher bereits vor Beginn der EPO-Behandlung aufzufüllen.

Zur Untersuchung des Eisenstoffwechsels werden in der klinischen Chemie die Konzentration von Eisen im Blut, und die Eisenbindungskapazität bestimmt. Es sollten immer beide Tests durchgeführt werden, da der Bezug ihrer Meßergebnisse zueinander wichtig ist. Gewöhnlich liegen die normaleu Serumeisenspiegel bei Männern zwischen 75 und 150 µg/dl und bei Frauen zwischen 60 und 140 µg/dl. Die totale Eisenbindungskapazität beträgt zwischen 250 und 450 µg/dl. Der Serumeisenspiegel schwankt im Tagesverlauf Er ist erniedrigt bei Eisenmangel und bei Anämien im Rahmen chronischer Erkrankungen. Er ist erhöht bei Hämolyse und bei Syndromen mit Eisenüberladung (z.B. Hämochromatose oder Hämosiderose). Patienten, die unter einer oralen Eisenmedikation stehen, können normale Eisen-Serumspiegel haben, obwohl eigentlich ein Eisenmangel bei ihnen vorliegt. Die totale Eisenbindungskapazität (= Transferrin x 2) ist erhöht beim Eisenmangel, dagegen aber erniedrigt bei Anämien im Verlauf von chronischen Erkrankungen.

Außerdem wird der Serumferritinspiegel bestimmt. Ferritin ist ein eisenspeicherndes Glykoprotein, von dem gewebetypische Isoferritine existieren und das im Serum immunologisch bestimmt werden kann, z.B. durch einen Radioimmunoassay (RIA) oder auch durch turbidimetrische Methoden. Der Ferritinwert ist ein Maß für die Eisenspeicherung im Gewebe. In den meisten Laboratorien liegt der Normalbereich zwischen 30 und 300 ng/ml, und der geometrische Mittelwert beträgt 88 bei Männern und 49 bei Frauen. Die Serum-Ferritinwerte stehen in enger Beziehung zum Eisengesamtvorrat des Körpers. Deshalb findet man erniedrigte Serum-Ferritinspiegel nur beim Eisenmangel. Erhöhte Spiegel findet man bei Eisenüberladung. Ebenfalls erhöhte Serum-Ferritinspiegel findet man bei Leberschäden oder in Assoziation mit manchen Neoplasien, wo Ferritine auch an Akute-Phase-Proteine gebunden sein können. Auch der Serumtransferrin-Rezeptor kann durch einen enzymverstärkten Immunabsorptionstest (enzyme-linked immunosorbent assay = ELISA) bestimmt werden. Dabei wird ein monoklonaler Antikörper gegen den löslichen Rezeptor verwendet. Der Referenzbereich liegt zwischen 0,5 - 3 mg/l. Der Spiegel ist erhöht bei geringem Mangel in den Eisenspeichern. Die Konzentrationen spezifischer Erythrozyten-Ferritine können bestimmt werden, um die Eisenspeicher zu charakterisieren, besonders dann, wenn das Setum-Ferritin bei Gewebeverletzungen oder durch Akute-Phase-Reaktionen nicht verwertbar ist.

Zur Untersuchung des Eisenstoffwechsels wird ferner auch der Erythrozyten-Ferritin-Spiegel bestimmt. In heparinisiertem Blut werden die Erythrozyten von den Leukozyten und Thrombozyten (die ebenfalls Ferritin enthalten) mittels Zentrifugation getrennt. Es folgen die Lyse der Erythrozyten und die immunologische Bestimmung des gespeicherten Ferritins. Das Erythrozyten-Ferritin spiegelt den Status der Eisenspeicher während der zurückliegenden 3 Monate wider (d.h. während der Lebenszeit eines Erythrozyten). Die Normalwerte liegen im allgemeinen zwischen 5 und 48 Attogramm (ag) pro Erythrozyt. Werte < 5 findet man bei Eisenmangelanämien, erhöhte Werte (oft > 100) bei Eisenüberladung (z.B. Hämochromatose). Ähnliche Aussagekraft hat die Bestimmung von Zinkprotoporphyrin.

Im folgenden wird die Erfindung an einem Ausführungsbeispiel näher erläutert:

11 Patienten mit gesicherter rheumatischer Arthritis und chronischer Entzündungsanämie (Hb bei Frauen < 12g/dl, bei Männern < 13 g/dl) wurden über 12 Wochen mit 150 IE EPO/kg Körpergewicht 2x wöchentlich s.c. und zusätzlich durch i.v-Gabe von Eisen-Succrose 200 mg/Woche bei Auftreten eines funktionellen Eisenmangels behandelt. Weitere 12 Wochen erfolgte eine Nachbeobachtungszeit.

Der Therapieverlauf wurde anhand der primären Wirksamkeitskriterien (Vitalitätsskala SF-36, Müdigkeitsskala (MAF), isometrische Muskelkraftmessung (MSI)) und der sekundären Wirksamkeitskriterien. nämlich der Krankheitsaktivitätsparameter DAS, (Desease Activity Score = Krankheits-Aktivitäts-Einteilung), RADAI (Rhymatoid Arthritis Desease Activity Index = Aktivitäts-Einteilung für Rheumatoide Arthritis) sowie Akute-Phase-Parameter CRP (C-reaktives Protein) untersucht:

Ergebnisse:

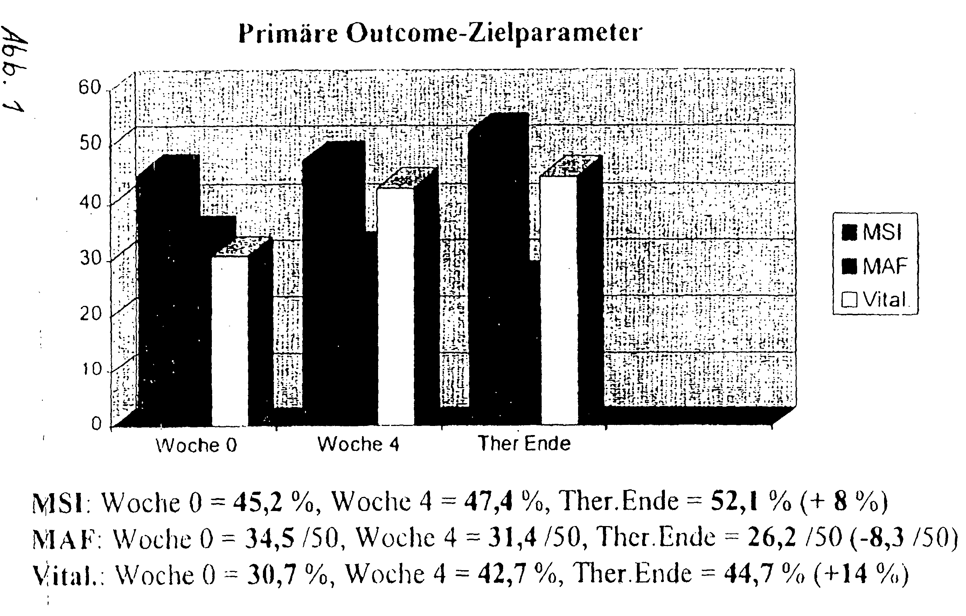

Wie der anliegenden Abb. 1 zu entnehmen ist, verlief die Therapie positiv. Hinsichtlich Muskelkraft war ein durchschnittlicher MSI-Anstieg von 8 % zu verzeichnen, die Vitalität stieg durchschnittlich um 14 % auf der Vitalitätsskala SF-36 und die Müdigkeit konnte durchschnittlich um 8,3 von 50 möglichen Punkten gesenkt werden.

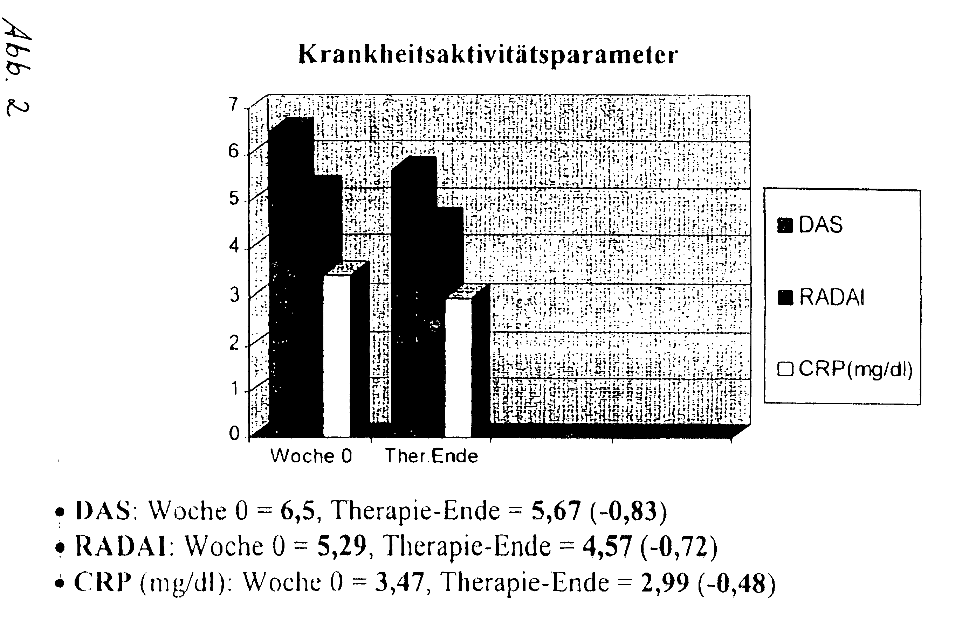

Der Abb. 2 können die Auswirkungen der Therapie auf die Krankheitsaktivitätsparameter DAS, RADAI und CRP entnommen werden.

So hatte DAS bei Therapie-Beginn einen Wert von 6,5, der am Therapie-Ende bei 5,67 lag. Der RADAI-Wert betrug bei Therapie-Beginn 5,29 und am Ende der Therapie 4,57 und CRP (mg/dl) war bei Therapie-Beginn 3,47 und am Ende der Therapie 2,99.

**Patentansprüche**

1. Verwendung von Erythropoietin und Eisenpräparaten in Form von

   a) einzelnen Darreichungsformen eines Erythropoietin-Präparates geeignet für die Einzeldosierung des Wirkstoffes in einer physiologisch wirksamen Menge und
   b) einem physiologisch verträglichen Eisenpräparat entsprechend einer äquivalenten Menge von 1 - 40 mg an Eisenionen

   zur Herstellung eines pharmazeutischen Kombinationspräparates zur Behandlung von rheumatischen Erkrankungen.

2. Verwendung nach Anspruch 1, **dadurch gekennzeichnet, daß** die einzelne Darreichungsform des Erythropoietin-Präparates den Wirkstoff in einer Menge von 250 - 20.000 U enthält.

3. Verwendung nach Anspruch 1 oder 2, **dadurch gekennzeichnet, daß** das pharmazeutische Kombinationspräparat zur Behandlung von entzündlichen Gelenkerkrankungen verwendet wird.

4. Verwendung nach einem der Ansprüche 1 - 3, **dadurch gekennzeichnet, daß** das Kombinationspräparat 500 - 10.000 U eines Erythropoietin-Präparates enthält.

5. Verwendung nach einem der Ansprüche 1 - 4, **dadurch gekennzeichnet, daß** das Eisenpräparat eine äquivalente Menge von 1 - 30 mg an Eisenionen enthält.

6. Verwendung nach einem der Ansprüche 1 - 5, **dadurch gekennzeichnet, daß** das Eisenpräparat eine äquivalente Menge von 3 - 20 mg an Eisenionen enthält.

7. Verwendung nach einem der Ansprüche 1 - 6, **dadurch gekennzeichnet, daß** das Eisenpräparat ein Komplex mit einem Molekulargewicht zwischen 30 000 - 100 000 D ist, vorzugsweise Fe(III)-Saccharat.

8.  Verwendung nach einem der Ansprüche 1 - 7, **dadurch gekennzeichnet, daß** das Eisenpräparat Fe(III)-Gluconat ist.

9.  Verwendung nach einem der Ansprüche 1 - 8 , **dadurch gekennzeichnet, daß** das Kombinationspräparat zusätzlich ein Präparat enthält, das eine suppressive Wirkung auf TNF-alpha besitzt, vorzugsweise ein Gluco-Corticosteroid-Präparat oder ein Präparat, das einen Antimetaboliten der Folsäure aufweist.

**Claims**

1.  Use of erythropoietin and iron preparations in the form of

    a) individual administration forms of an erythropoietin preparation suitable for the individual dosing of the active substance in a physiologically effective amount and
    b) a physiologically compatible iron preparation corresponding to an equivalent amount of 1-40 mg of iron ions

    for the production of a pharmaceutical combination preparation for the treatment of rheumatic diseases.

2.  Use according to claim 1, **characterized in that** the individual administration form of the erythropoietin preparation contains the active substance in an amount of 250-20,000 U.

3.  Use according to claim 1 or 2, **characterized in that** the pharmaceutical combination preparation is used for the treatment of inflammatory joint diseases.

4.  Use according to any one of claims 1-3, **characterized in that** the combination preparation contains 500-10,000 U of an erythropoietin preparation.

5.  Use according to any one of claims 1-4, **characterized in that** the iron preparation contains an equivalent amount of 1-30 mg of iron ions.

6.  Use according to any one of claims 1-5, **characterized in that** the iron preparation contains an equivalent amount of 3-20 mg of iron ions.

7.  Use according to any one of claims 1-6, **characterized in that** the iron preparation is a complex with a molecular weight between 30,000-100,000 D, preferably Fe(III) saccharate.

8.  Use according to any one of claims 1-7, **characterized in that** the iron preparation is Fe(III) gluconate.

9.  Use according to any one of claims 1-8, **characterized in that** the combination preparation additionally contains a preparation which has a suppressive action on TNF-alpha, preferably a gluco-corticosteroid preparation or a preparation which has an antimetabolite of folic acid.

**Revendications**

1.  Utilisation de préparations d'érythropoïétine et de fer sous forme de

    a) formes d'administration séparées d'une préparation d'érythropoïétine appropriées au dosage unique de l'actif dans une quantité physiologiquement efficace et
    b) une préparation de fer physiologiquement acceptable correspondant à une quantité équivalente allant de 1 à 40 mg d'ions fer

    pour la production d'une préparation combinée pharmaceutique pour le traitement des maladies rhumatismales.

2.  Utilisation selon la revendication 1, **caractérisée en ce que** la forme d'administration séparée de la préparation d'érythropoïétine comprend l'actif en une quantité de 250 à 20 000 U.

3. Utilisation selon la revendication 1 ou 2, **caractérisée en ce que** l'on utilise la préparation combinée pharmaceutique pour le traitement des maladies inflammatoires des articulations.

4. Utilisation selon l'une quelconque des revendications 1 à 3, **caractérisée en ce que** la préparation combinée comprend de 500 à 10 000 U d'une préparation d'érythropoïétine.

5. Utilisation selon l'une quelconque des revendications 1 à 4, **caractérisée en ce que** la préparation de fer comprend une quantité équivalente de 1 à 30 mg d'ions fer.

6. Utilisation selon l'une quelconque des revendications 1 à 5, **caractérisée en ce que** la préparation de fer comprend une quantité équivalente de 3 à 20 mg d'ions fer.

7. Utilisation selon l'une quelconque des revendications 1 à 6, **caractérisée en ce que** la préparation de fer est un complexe ayant un poids moléculaire entre 30 000 et 100 000 D, de préférence le saccharate de Fe (III).

8. Utilisation selon l'une quelconque des revendications 1 à 7, **caractérisée en ce que** la préparation de fer est du gluconate de Fe (III).

9. Utilisation selon l'une quelconque des revendications 1 à 8, **caractérisée en ce que** la préparation combinée comprend en plus une préparation qui possède une action suppressive sur le TNF-alpha, de préférence une préparation de glucocorticostéroïde ou une préparation qui présente un antimétabolite de l'acide folique.

**Abb. 1**

## Primäre Outcome-Zielparameter

MSI: Woche 0 = **45,2** %, Woche 4 = **47,4** %, Ther.Ende = **52,1** % (+ **8** %)

MAF: Woche 0 = **34,5** /50, Woche 4 = **31,4** /50, Ther.Ende = **26,2** /50 (**-8,3** /50)

Vital.: Woche 0 = **30,7** %, Woche 4 = **42,7** %, Ther.Ende = **44,7** % (**+14** %)

*Abb. 2*

## Krankheitsaktivitätsparameter

- **DAS**: Woche 0 = **6,5**, Therapie-Ende = **5,67** (**-0,83**)
- **RADAI**: Woche 0 = **5,29**, Therapie-Ende = **4,57** (**-0,72**)
- **CRP** (mg/dl): Woche 0 = **3,47**, Therapie-Ende = **2,99** (**-0,48**)